## Europäisches Patentamt
## European Patent Office
## Office européen des brevets

(11) Publication number: **0 003 407**
**B1**

(12) # EUROPEAN PATENT SPECIFICATION

(45) Date of publication of patent specification: **30.09.81**

(21) Application number: **79300079.5**

(22) Date of filing: **17.01.79**

(51) Int. Cl.³· **A 61 K 33/30,
A 61 K 31/43,
A 61 K 31/545, //
(A 61 K 33/30, 31/43, 31/20,
31/23, 31/545),
(A 61 K 31/43, 31/20, 31/19,
31/23, 31/315)**

(54) **Pharmaceutical and dietary composition comprising gamma-linolenic acids**

(30) Priority: **23.01.78 GB 264278
19.04.78 GB 1548178
17.08.78 GB 3368278
24.10.78 GB 4176178**

(43) Date of publication of application
**08.08.79 Bulletin 79/16**

(45) Publication of the grant of the European patent:
**30.09.81 Bulletin 81/39**

(84) Designated Contracting States:
**BE CH DE FR GB IT LU NL SE**

(56) References cited:
**FR - A - 2 272 684**

(73) Proprietor: **EFAMOL LIMITED
71/74 Mark Lane
London E.C.3 (GB)**

(72) Inventor: **Horrobin, David Frederick
110 Pine Avenue West
Montreal (CA)**

(74) Representative: **Caro, William Egerton, et al
J. MILLER & CO.
Lincoln House
296-302 High Holborn
London WC1V 7JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Pharmaceutical and dietary composition comprising gamma–linolenic acids

This invention relates to compositions for the treatment of various diseases and disorders primarily, but not exclusively, in the field of human medicine.

Considerable interest has been shown in recent years in the use of prostaglandin (PG) precursors in medicine.

For various reasons it is not practical to administer naturally-occurring prostaglandins such as PGE 1 and PGE 2 to patients. Consequently, considerable attention has focussed on the use of prostaglandin precursors including linoleic acid (9,12-octadecadienoic acid), $\gamma$-linolenic acid (6,9,12-octadecatrienoic acid) and dihomo-$\gamma$-linolenic acid (5,8,11-eicosatrienoic acid), conversion in the body being believed to be as follows:

Linoleic acid

$\downarrow$

$\gamma$-Linolenic acid

$\downarrow$

Esterified reserves $\rightleftharpoons$ Dihomo-$\gamma$-linolenic acid $\searrow$

$\downarrow$ $\qquad$ Prostaglandins of the 1 series

Esterified reserves $\rightleftharpoons$ Arachidonic acid (5,8,11,14-eicosatetraenoic acid)

$\downarrow$

Prostaglandins of the 2 series

Prior art within this general area includes the following patents and papers.

(i) U.S. Patents Nos. 3 993 775 (issued November 23rd, 1976) and 4 058 594 (issued November 15th, 1977) of John Williams, which describe a method of providing an immuno-suppressive effect in a patient undergoing organ or tissue transplant or suffering from multiple sclerosis comprising administration of a daily dosage of from 5 mg to 3 g of $\gamma$-linolenic acid or dihomo - $\gamma$ - linolenic acid or a functional derivative thereof.

(ii) British Patent Specification No. 1 082 624, published September 6th, 1967, (Calmic Limited), which discloses effectiveness of $\gamma$-linolenic acid in the treatment of vascular diseases.

(iii) McCormack, Neil and Sim (The Lancet, page 308, September 3rd, 1977) who describe preliminary work on the use of an oil containing a mixture of linolenic acid and $\gamma$-linolenic acid (as triglycerides) in the treatment of rheumatoid arthritis.

(iv) Sim and McCraw (Thrombosis Research Volume 10, pages 385—397, 1977), who describe activity of the methyl esters of $\gamma$-linolenic acid and dihomo-$\gamma$-linolenic acid in vitro and in vivo on blood platelet function in non-human primates and in man.

(v) French Specification No. 2 272 684, where there is a proposal to use various unsaturated fatty acids including 'linolenic' acid (presumably $\alpha$-linolenic acid) to enhance the effect of antibiotics.

The present inventor has discovered a number of new applications of $\gamma$-linolenic acid and dihomo - $\gamma$ - linolenic acid in therapy, in conjunction with zinc and/or $\beta$-lactam antibiotics. These are now discribed in turn.

SCHIZOPHRENIA

In the Lancet, page 936, April 30, 1977 the present inventor has suggested that schizophrenia is a prostaglandin deficiency disease. Schizophrenia is not a disorder which would suggest the use of immuno-suppressive drugs. The specific suggestion was made that arachdonic acid, known to be a precursor of prostaglandins of the 2 series should alleviate schizophrenia.

As a result of further research, the present inventor now believes that schizophrenia is due not to a deficiency of 2 series PG's but rather to a deficiency of PGE 1 and other PG's of the 1 series, of which arachidonic acid is not a precursor, or an imbalance in the normal ratio of 1 series and 2 series PG's.

This has led to the realisation that the materials which should be used to stimulate the natural production of 1 series PG's in the treatment of schizophrenia should include $\gamma$-linolenic acid and/or dihomo - $\gamma$ - linolenic acid, either or both of which may be used in association with linoleic acid and if desired other fat acids. Although these substances are 2 series PG precursors (via arachidonic acid) as well as 1 series PG precursors, this is not deleterious to their use, although one may

2

require to use relatively higher amounts of precursors than would be the case if only 1 series PG's were being biosynthesized.

## SKIN DISORDERS

A further area where the administration of 1 series PG precursors is indicated is in the treatment of psoriasis and other human skin disorders such as acne, dandruff, eczema and hair loss (other than that due to inherited male pattern baldness).

The physiological basis for these treatments is not understood in detail but it is believed by the present inventor that conditions such as psoriasis, dandruff, eczema and hair loss are related to each other by common, or at least related, defects in 1 series PG precursor metabolism, expressing themselves in various ways in different individuals. Experimental evidence of a relation is discussed below in the section on veterinary application of the invention.

## OBESITY

A current technique for the treatment of obesity involves the administration of linoleic acid, generally in the form of vegetable oils such as sunflower oil and/or corn oil. In order to be effective, these current dietary approaches to the treatment of obesity require the intake of other fats in the diet to be substantially reduced. In the body, linoleic acid is converted as described earlier and the present inventor believes that the beneficial effect of administration of linoleic acid is due to enhancement of 1 series PG production and in particular of PGE 1, this substance causing a metabolic shift increasing appetite and reducing weight. However, the presence of other fats in the diet interferes with the conversion of linolenic acid to $\gamma$-linolenic acid and thus reduces the effectiveness of the treatment.

What is now proposed is administration of materials including $\gamma$-linolenic acid and/or dihomo - $\gamma$ -linolenic acid or derivatives, as effective in the treatment of obesity, even when other fats are present in the diet.

## MENSTRUAL DISORDERS

Menstrual disorders are not uncommon and while they do not usually require clinical treatment they are often a cause of distress or discomfort. Such disorders include: extended periods of blood loss, sometimes for as long as 9 days or more, especially when using intra-uterine contraceptive devices; excessive blood loss during menstrus, which is again often associated with the use of intra-uterine contraceptive devices; so-called "period pains"; premenstrual swelling associated with excessive fluid retention; and irregular menstrual cycle lengths.

The present inventor has now surprisingly found that the administration of materials including $\gamma$-linolenic acid and/or dihomo - $\gamma$ - linolenic acid or derivatives causes a significant reduction in some or all of the above mentioned menstrual disorders.

In tests which have been effected by the present inventor, it has been found that women previously exhibiting excessive periods of blood loss during menstrus experienced a reduction in this period to 3 to 5 days on treatment according to the invention. In addition these tests have shown that a reduction in amount of blood loss, period pains and premenstrual swelling and a stabilisation of menstrual cycle lengths, may be achieved.

The physiological explanation for the efficacy of the treatment is not fully understood. However, whilst not wishing to be bound by theoretical considerations, the inventor has noted that these types of menstrual disorders are often associated with obesity, which appear in at least some subjects to be due to a deficiency in essential fatty acids.

## USE OF ZINC

As has been mentioned above, $\gamma$-linolenic acid and dihomo $\cdot \gamma \cdot$ linolenic acid function as precursors for both 1 and 2 series PG's. The present inventor specifically believes it advantageous if the biosynthesis of 1 series PG's can be effected preferentially to that of 2 series PG's in conditions, not merely schizophrenia and the other applications discussed in some detail herein, but also the conditions treated according to the prior art discussed in which 1 series PG imbalances or lack need to be corrected.

Without restriction to the theory, the present inventor believes that zinc tends to stimulate the biosynthesis of 1 series PG's and specifically that it potentiates mobilisation of esterified reserves of dihomo - $\gamma$ - linolenic acid. This enables one to use zinc conjointly with $\gamma$-linolenic acid and/or dihomo - $\gamma$ - linolenic acid. The presence of arachidonic acid or any other material tending to oppose the PG 1 enhancing effect is to be avoided.

## USE OF $\beta$-LACTAM ANTIBIOTICS

The use of $\gamma$-linolenic or other acids and derivatives with $\beta$-lactam antibiotics is also valuable. The present inventor believes that the reason for the effectiveness of the antibiotics is that, as he believes with zinc, they enhance utilisation of ester reserves of dihomo $\gamma$ - linolenic acid. Whether or not this is so, and no restriction to the theory is intended, zinc and antibiotics do appear to have parallel effects in treating all the conditions when used with the $\gamma$-linolenic or other acids and derivatives.

It is also possible and has been found valuable to use both zinc and $\beta$-lactam antibiotic conjointly with the $\gamma$-linolenic acid, dihomo - $\gamma$ - linolenic acid or derivatives.

## FORMAL STATEMENT OF INVENTION

The invention thus specifically provides a pharmaceutical or dietary composition comprising $\gamma$-linolenic acid or physiologically functional derivative thereof and/or dihomo $\cdot \gamma \cdot$ linolenic acid or physiologically functional derivative thereof and a conjoint amount of a $\beta$-lactam antibiotic alone or in an acceptable pharmaceutical or dietary vehicle.

Alternatively if it is not desired to have compositions comprising both the antibiotic (or the antibiotic and zinc) and the $\gamma$-linolenic or other acid or derivative, packs may be prepared comprising the active materials presented for separate administration or two together and one separately in the appropriate relative amounts, and such packs are within the pur view of the invention.

## AMOUNTS OF $\gamma$-LINOLENIC AND OTHER ACIDS

A preferred daily dosage for an adult (weight ca 75 kg) is from 0.05 or 0.1 up to 1 2, 5 or even 10 g as required of $\gamma$-linolenic acid or equivalent weight (calculated as $\gamma$-linolenic acid) of physiologically functional derivative thereof. Amounts may in particular be 0.1 to 1.0 g daily In place of, or in addition to, $\gamma$-linolenic acid, one may use dihomo $\cdot \gamma$ - linolenic acid or a physiologically functional derivative thereof, in amounts equivalent in molar terms to $\gamma$-linolenic acid and calculated as such. This dosage can for example be taken as a single dose or divided into 2, 3, or 4 subdivisions thereof as convenient.

## FORMS AND SOURCES OF $\gamma$-LINOLENIC AND OTHER ACIDS

Convenient physiologically functional derivatives of $\gamma$-linolenic acid and dihomo $\gamma$ linolenic acid for use for all the purposes described include the $C_1$—$C_4$ alkyl (e.g. methyl and ethyl) esters and the glycerides of the acids.

If desired, pharmaceutical compositions may be produced for use in the invention by associating natural or synthetic $\gamma$-linolenic acid (or a physiologically functional derivative thereof) and/or dihomo $\cdot \gamma$ - linolenic acid (or a physiologically functional derivative thereof) as such, with an acceptable pharmaceutical vehicle. It will however generally be convenient to incorporate the $\gamma$-linolenic acid into compositions in the form of an available oil having a high $\gamma$-linolenic acid content.

At the present time known natural sources of oils having a high $\gamma$-linolenic acid content are few (there are no known natural sources of significant amounts of dihomo - $\gamma$ - linolenic acid).

One source of oils currently available is the seed of Evening Primrose species such as *Oenothera biennis L.* and *Oenothera lamarckiana,* the oil extract therefrom containing $\gamma$-linolenic acid and linolenic acid in the form of their glycerides together with other glycerides. Another source of $\gamma$-linolenic acid is the seed of Borage species such as *Borago*

*officinalis* which, though its current yield per acre is low, provides a richer source of $\gamma$-linolenic acid than Oenothera oil. Recent studies on fungi which can be cultivated by fer mentation promise a fungal oil source.

The seed oil extracts referred to above can be used as such or can if desired be fractionated to yield an oily composition containing the triglycerides of $\gamma$-linolenic acid and linoleic acid as the only fatty acid components the $\gamma$-linolenic acid conten† being a major proportion Seed oil extracts appear to have a stabilising effect upon any dihomo $\gamma$ linolenic acid or physiologically functional derivative thereof incorporated therein

## AMOUNTS AND FORMS OF ZINC

Based on present evidence, a suitable daily dosage of zinc for an adult (weight ca 75 kg) is 2.5—800 mg preferably 10—200 mg and advantageously 10—80 mg zinc daily, with $\gamma$-linolenic acid or other acid or equivalent in the amounts previously discussed. The 10—80 mg zinc is approximately 0.125—1.0 mg/kg adult body weight. In view of the conjoint effect of the zinc preferred amount of $\gamma$-linolenic or other acid or equivalent are less than when zinc is not present, advantageously 0.1 to 1.0 g daily. As before the dosage can be taken as a single dose or divided into 2, 3 or 4 subdivisions thereof.

The zinc should be administered in a form in which it is readily taken up in vivo. Ordinarily this will indicate the use of a zinc salt of a mineral or organic acid, said salt being physiologically acceptable at the given dosage. Some zinc salts which would be contra-indicated at higher dosages may be satisfactory for present purposes at the dosages indicated above. Useful salts include zinc sulphate and zinc gluconate and in particular zinc oleate, $\gamma$-linoleniate and dihomo - $\gamma$ - linolenate, and zinc oxide may also be employed. It is also possible to administer the zinc in chelated form. In any event, the preferred amounts of zinc are as stated above (the quantities given being calculated as zinc metal).

## KINDS AND AMOUNTS OF ANTIBIOTIC

$\beta$-lactam antibiotics which may be used according to the present invention, are conveniently any of the known penicillin and cephalosporin antibiotics (including semisynthetic antibiotics) such as, for example, penicillin G, penicillin N, penicillin V, cephalexin, cephalothin, ampicillin, amoxycillin, cloxacillin and cephalogylcin. Any of these may be used in the form of their physiologically functional non-toxic derivatives, for example alkali metal salts e.g. sodium and potassium salts, and salts with organic bases, and reference to an antibiotic herein (including the claims) includes reference to such derivatives.

The antibiotic is preferably administered in daily dosages of for example 0.5 to 3.0 g per day in patients of average weight. Such daily dosages may conveniently be divided into for example, two, three or four equal doses to be administered two, three or four times daily respectively.

## SPECIFIC INDICATIONS IN SCHIZOPHRENIA

In severely disturbed patients it may be desirable to additionally administer conventional tranquilizers in addition to regular treatment with the compositions of the invention, but this is only required when such patients experience extreme agitation, insomnia or hallucinations.

The use of penicillins in the long term treatment of schizophrenia is especially desirable in view of the known relative absence of side effects of these drugs. Thus, penicillin has been administered for many years to patients having rheumatic heart disease in order to prevent streptococcal infections, and there is virtually no evidence of long term toxicity.

Care should of course be taken to ensure that the patient is not allergic to the drug of choice. With respect to the known ability of penicillins to produce reactions in some patients due to penicillin hypersensitivity, there is evidence to suggest that schizophenics have a reduced incidence of allergic reactions and more particularly of penicillin hypersensitivity. Thus, the problem, usually associated with penicillin antibiotic therapy, of hypersensitization in a small number of patients, is not quite so important in the treatment of schizophrenia using penicillins.

A valuable benefit of the present invention is that the hitherto extensively used chemotherapeutic agents of schizophrenia have been associated with a tranquilizing activity, with the result that the use of these drugs in therapy is combined with an often undesired heavy sedation of the patient. Also such drugs may be responsible for the production of irreversible damages in up to 70% of patients to those parts of the brain which control movement. Avoidance or substantial avoidance of the use of these drugs is thus of great value.

## DIETARY COMPOSITIONS

The invention is chiefly described in terms of pharmaceutical compositions, but it will be understood that the $\gamma$-linolenic and other acids, being in the nature of dietary supplements, could if available at an economic price be incorporated in a dietary margarine or other foodstuff; such foodstuffs, referred to herein as dietary compositions, are within the purview of the invention.

## VETERINARY APPLICATIONS

It will be understood that where a disorder of the kind calling for treatment in animals arises, the invention while described primarily in terms of human medicine and treatment is equally applicable in the veterinary field.

Thus for example domestic cats have an unusual dietary requirement in essential fatty acids, being apparently unable to convert linoleic acid to $\gamma$-linolenic and dihomo · $\gamma$ - linolenic acid to arachidonic acid. They are liable to a group of related skin conditions with hair loss, dandruff, scaling, pruritis, easy breakdown of the skin with rubbing or scratching, and defective healing, all of which can also be produced experimentally by an EFA (essential fatty acid) deficient diet, evidencing their related nature. Similar conditions can be produced experimentally in other animals, with skin lesions similar to eczema and psoriasis. Feeding of $\gamma$- or dihomo - $\gamma$ - linolenic acid is effective in reversing the conditions, including, perhaps surprisingly, those in cats. This indicates, in view of the arachidonic acid block, that the conditions are indeed, as the present inventor believes also for the human skin conditions discussed above, related to 1 series PG deficiencies. The spontaneous conditions observed in cats are for example relieved by giving 0.5 g of Oenothera oil and zinc 20 mg as sulphate, per day five days a week.

## PHARMACEUTICAL PRESENTATION

The compositions according to the invention are conveniently in a form suitable for oral, rectal, parenteral or topical administration in a suitable pharmaceutical vehicle, as discussed in detail for example in U.K. Patent Specification No. 1 082 624 and in any case known generally according to the type of preparation. Thus for example tablets, capsules, ingestible liquid or powder preparations, creams and lotions for topical application, or suppositories, can be prepared as required.

Advantageously on antioxidant such as $\alpha$-tocopherol is incorporated into the preparations. $\alpha$-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions according to the invention:

### Examples

Pharmaceutical compositions containing a unit does of an oil extract from the seeds of *Oenothera biennis L.* optionally with methyl dihomo - $\gamma$ - linolenate and with zinc in suitable form and/or penicillin V are prepared by encapsulation of the natural oil in soft gelatin capsules manufactured by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| Palmitate | 6.15 |
|---|---|
| Stearate | 1.6 |
| Oleate | 10.15 |
| Linoleate | 72.6 |
| $\gamma$-Linolenate | 8.9 |

As preservative, $\alpha$-tocopherol is added to the oil in a concentration of 0.1%.

Gelatin capsules containing oil extracts prepared as described above, each having the following contents of active ingredients (0.5 g oil extract = ca 0.045 g $\gamma$-linolenic acid), are prepared in conventional fashion. The zinc may conveniently be incorporated as zinc oleate made by the method disclosed in Monatschrift **42** 287 (1921), and similar methods may be applied to make for example zinc $\gamma$-linolenate if desired

### Example 1

| Oil extract | 0.5 g |
|---|---|
| Zinc sulphate | 10 mg |

Two capsules may be administered thrice daily in the treatment of schizophrenia or menstrual disorders, giving a daily dose of $\gamma$-linolenic acid of ca 0.27 g. Similar capsules with 20 mg zinc sulphate may be administered in the treatment of acne, psoriasis, eczema, dandruff and loss of hair, or of obesity.

### Example 2

| Oil extract | 0.5 g |
|---|---|
| Methyl dihomo - $\gamma$ - linolenate | 10 mg |
| Zinc sulphate | 10 mg |

Two capsules may be administered thrice daily in the treatment of schizophrenia.

### Example 3

| Oil extract | 0.5 g |
|---|---|
| Penicillin V | 0.25 g |

### Example 4

Two capsules may be administered thrice daily in the treatment of schizophrenia.

| Oil extract | 0.5 g |
|---|---|
| Penicillin V | 0.25 g |
| Zinc sulphate | 10 mg |

Two capsules may be administered thrice daily in the treatment of schizophrenia.

### Example 5

| Oil extract | 0.5 g |
|---|---|
| Methyl dihomo-$\gamma$-linolenic | 10 mg |
| Penicillin V | 0.25 g |
| Zinc sulphate | 10 mg |

Two capsules may be administered thrice daily in the treatment of schizophrenia.

### Example 6

| Oil extract | 0.5 g |
|---|---|
| Methyl dihomo-$\gamma$-linolenate | 10 mg |

Two capsules may be administered twice daily in the treatment of menstrual disorders.

## EVIDENCE OF EFFICACY

The conditions are considered in turn.

## SCHIZOPHRENIA — Use of Oenothera Oil and Penicillin

A female patient aged 50 who had suffered for 20 years from severe schizophrenia and was aggressive, paranoid and hypochondriacal in spite of conventional drug treatment with haloperidol (10 mg three times a day) plus flupenthixol decanoate (40 Mg/month), was given Oenothera oil (2 x 0.6 ml capsules four times a day) and penicillin V (250 mg four times a day). There was some initial nausea and headache but after two weeks hypochondriacal delusions ceased and after six weeks paranoid delusions, aggressiveness and incongruity of affect has also disappeared. Further, 6 kg in weight were lost in the course of 16 weeks in spite of a regular diet.

A further, male patient of 31 has suffered from severe schizophrenic illness for 12 years and had been an in-patient for 7 years, aggressive, hearing voices, of wild staring appearance and not speaking spontaneously to others. He had been receiving fluphenazine decanoate 75 mg every two weeks, benzhexol 5 mg three times a day and supplementary chlorpromazine as required. He was taken off these drugs and given Oenothera oil and penicillin as above for one month and an increase to 3 capsules qds in the oil thereafter. Over a period of six months he became co-operative, not easily upset by fellow patients, without aggressions, speaking spontaneously and appropriately to others, and with almost normal affect. His BPRS (Brief Psychiatric Rating Score) score dropped from 44 to 21 over the period.

Four other severe chronic schizophrenics controlled by phenothiazines were withdrawn from them and given the Oenothera oil and penicillin. The condition of each was maintained without the side effects of the other drugs.

Use of Oenothera oil, Penicillin and Zinc

Preliminary trials with a small group of similar patients to those in the previous trials hav been promising on the following:

Oenothera oil 6 or 8 × 0.6 ml capsules/day

Penicillin 250 mg qds

Zinc, as sulphate 20 to 40 mg/day

## SKIN DISORDERS

Acne and psoriasis are two common and intractable conditions that have shown favourable results with treatment according to the invention.

A group of sufferers from severe acne, of 7 young men, receive Oenothera oil 0.6 ml + zinc sulphate 20 mg, 6 capsules daily. All showed improvement in terms of reduction both in the number of inflamed facial pustules and in sebum production rate, over a period of 4 to 6 weeks.

After three months all the subjects showed a very substantial improvement, most being essentially clear of pustules.

A group of 4 subjects with psoriasis was given similar treatment. In all, scaliness and itching were reduced. In no case was there a full cure, but psoriasis is a particularly intractable condition in which even a modest improvement is clinically significant.

No clinical trials have been done on hair loss, but in a group of 30 laboratory rats maintained on a zinc deficient diet, hair loss was reversed by feeding of Oenothera oil with zinc, the effect being greater than when zinc was given alone.

Preliminary results with eczema using Oenothera oil and zinc together have given favourable indications, as found with psoriasis. Dandruff also responded favourably on two individuals otherwise wholly healthy.

## OBESITY

Thirty-eight healthy subjects initially took Oenothera oil in 0.6 ml capsules for 6—8 weeks, thirty-four of them at 6 capsules a day and four at eight capsules a day, while continuing to eat their normal diets.

Twenty-two of the subjects, all taking 6 capsules a day, were initially within 10% of their ideal body weight according to the standard life tables. None gained or lost more than 2 kg.

The other sixteen subjects were all more than 10% above their ideal body weight. Of this group, two women and three men showed no change in weight. They were taking 6 capsules a day. Six women and five men lost weight, as follows:

| Initial mean weight kg | 74.55 ± 7 94 (S.D.) |
| Final mean weight kg | 70.42 ± 6.52 (p ± 0.5, paired t test) |

Of the subjects who lost weight, four who were taking 8 capsules a day lost 8.2, 10.0, 10.9 and 12.7 kg respectively. All the other subjects who lost weight were taking 6 capsules a day.

These results indicate that overweight individuals, but not those of normal weight, have a good chance of losing weight by simple inclusion of Oenothera oil in the diet, and that the weight loss is dose related.

No adverse effects were observed. The intake of 6 capsules a day, 3.6 ml of the oil, is equivalent to ca 0.27 g of $\gamma$-linolenic acid; of 8 capsules a day, 4.8 ml of the oil, ca 0.36 g of $\gamma$-linolenic acid.

Preliminary results indicate improvement in terms of weight loss and proportion of subjects responding, when capsules containing 20 mg zinc sulphate in addition to the oil are used.

## MENSTRUAL DISORDERS

A group of 15 women of reproductive age was treated, four of whom were using an intrauterine contraceptive device and all of whom were suffering from prolonged menstrus of 8 to 12 days with excess blood loss. Of these women, 5 also suffered from the premenstrual syndrome of depression, pain and fluid retention.

Initial administration of Oenothera oil 6 × 0.6 ml capsules daily over a period of several months consistently reduced the duration of blood loss to 3 to 5 days with concomitant reduction in the amount. The symptoms of the premenstrual syndrome in those women showing it became mild, or in one instance disappeared entirely and stayed gone for 3 full cycles, up to the end of the trial.

Preliminary results indicate a further improved effect of oil capsules administered with 20 mg zinc sulphate.

## USE OF ZINC

Substantial clinical results are not at present available on all the conditions for which the use of zinc is proposed, but the present inventor believes, without wishing to be limited to the theory, that at the root of all the conditions lies a fault in prostaglandin metabolism whereby PG's of the 1 series are lacking or their balance with 2 series PG's is upset. From evidence such as that listed below the inventor believes that zinc increases formation of 1 series PG's selectively, apparently by mediating the mobilisation from ester resource of dihomo - $\gamma$ - linolenic acid.

Thus zinc is indicated in all the conditions described herein, as favouring 1 series PG synthesis specifically from administered $\gamma$-linolenic acid and related materials.

In one group of experiments the test preparation was the isolated superior mesenteric vascular bed, taken from male rats as for example described in the Canadian J. Physiol Pharmacol 54:357, 1976. The perfusion flow rate was at a constant value between 3 to 4 ml/min., pressure 25 to 30 mm Hg, using Krebs bicarbonate buffer containing in nM 150 Na, 4.3 K, 1.0 Mg, 2.5 Ca, 1 7 phosphate, 25 bicarbonate and 11 1 glucose.

Prior to testing the basic vasoconstrictive effect of norepinephrine, as the bitartrate, in successive 10 ng amounts was established, as the amplitude of a transient rise of about 1 min in the perfusion pressure.

Zinc, as the sulphate, was then added to the perfusion buffer at successive concentrations and the norepinephrine response measured after 15 minutes at each.

The following results were obtained

| Zinc concentration ($\mu$g/ml) | Response as % of basic level |
|---|---|
| 0.1 | 112 |
| 0.2 | 118 |
| 0.4 | 130 |
| 0.8 | 138 |

In the presence of 50 $\mu$g/ml of indomethacin, a known blocking agent for PG synthesis, used with 10 ng/ml PGE to give apparently normal vascular reactivity, the zinc had no effect on the norepinephrine response.

Similar test with dihomo $\cdot \gamma$ linolenic acid and PGE 1 gave respective rises up to a maximum of 130% of the basic response at 50 ng/ml of the acid and a maximum of 150% of the basic response at $2.8 \times 10^{-11}$M PG.

The results show that zinc gives responses like those of dihomo - $\gamma$ - linolenic acid and of PGE 1, responses moreover which are not given when PG synthesis is blocked and PGE 2 supplied, and thus that conditions treated with $\gamma$-linolenic acid (and thus effectively with dihomo - $\gamma$ - linolenic acid) may be enhanced in the direction of 1 series PG synthesis by the addition of zinc.

## USE OF ANTIBIOTICS

On tests carried out as above, both penicillin V and penicillin G have given responses similar in kind and degree to those given for zinc, supporting further the inventor's belief that $\beta$-lactam antibiotics are of value in all other conditions treated according to the invention, in similar way to the action of zinc, and as evidence in the results on schizophrenia.

## Claims

1. A pharmaceutical or dietary composition comprising $\gamma$-linolenic acid do physiologically functional derivative thereof and/or dihomo $\cdot \gamma \cdot$ linolenic acid or physiologically functional derivative thereof and a conjoint amount of a $\beta$-lactam antibiotic, alone or in an acceptable pharmaceutical or dietary vehicle.

2. A composition according to claim 1 wherein the antibiotic is a natural or semisynthetic penicillin or cephalosporin antibiotic.

3. A composition according to claim 2, wherein the antibiotic is selected from penicillin G, penicillin N, penicillin V, cephalothin, ampicillin, amoxycillin, cloxacillin, cephhalexin and cephaloglycin.

4. A composition according to claims 1, 2 or 3 additionally comprising physiologically assimilable zinc.

5. A pharmaceutical or dietary composition for use in the treatment of schizophrenia, comprising $\gamma$-linolenic acid or physiologically functional derivative thereof and/or dihomo - $\gamma$ - linolenic acid or physiologically functional derivative thereof and a conjoint amount of a material influencing the 1-series/2-series PG balance in the body in favour of 1-series PG's, alone or in an acceptable pharmaceutical or dietary vehicle.

6. A pharmaceutical or dietary composition for use in the treatment of psoriasis, acne, dandruff, eczema, or hair loss (other than that due to inherited male pattern baldness), comprising $\gamma$-linolenic acid or physiologically functional derivative thereof and/or dihomo $\cdot \gamma \cdot$ linolenic acid or physiologically functional derivative thereof and a conjoint amount of a material influencing the 1-series/2-series PG balance in the body in favour of 1-series PG's, alone or in an acceptable pharmaceutical or dietary vehicle.

7. A pharmaceutical or dietary composition for use in the treatment of obesity, comprising $\gamma$-linolenic acid or physiologically functional derivative thereof and/or dihomo - $\gamma$ - linolenic acid or physiologically functional derivative there together with a conjoint amount of a material influencing the 1-series/2-series PG balance in the body in favour of 1-series PG's, alone or in an acceptable pharmaceutical or dietary vehicle.

8. A pharmaceutical or dietary composition for use in the treatment of menstrual disorders including pre-menstrual syndromes and excessive blood loss in or duration of menstrus, comprising $\gamma$-linolenic acid or physiologically functional derivative thereof and/or dihomo $\cdot \gamma$ - linolenic acid or physiologically functional derivative thereof and a conjoint amount of a material influencing 1-series/2-series PG balance in the body in favour of 1-series PG's, alone or in an acceptable pharmaceutical or dietary vehicle.

9. A composition according to any of claims 5 to 8 wherein said conjoint material is physiologically assimilable zinc.

10 A composition according to any of claims 5 to 8 wherein said conjoint material is a β-lactam antibiotic.

11 A composition according to claim 10 wherein the antibiotic is a natural or semisynthetic penicillin or cephalosporin antibiotic.

12 A composition according to claim 10, wherein the antibiotic is selected from penicillin G, penicillin N, penicillin V, cephalothin, ampicillin, amoxycillin, cloxacillin, cephalexin and cephaloglycin.

13 A composition according to any of claims 10 to 12 additionally comprising physiologically assimilable zinc.

14. A composition according to any preceding claim, wherein the physiologically functional derivative of $\gamma$-linolenic acid or dihomo $\gamma$ linolenic acid is a methyl or ethyl ester or glyceride thereof.

15. A composition according to any preceding claim wherein the $\gamma$-linolenic acid is present in the form of the oil of the seed of Oenothera biennis L Oenothera lamarckiana, or other Evening Primrose species, or a fraction thereof.

16. A composition according to any preceding claim wherein the $\gamma$-linolenic acid is present in the form of the oil of the seed of Brago officinalis or other borage species, or a fraction thereof.

17 A composition according to any preceding claim specifying the presence of zinc, wherein the zinc is present as a salt of a mineral or organic acid (and in particular zinc oleate, zinc $\gamma$-linolenic or zinc dihomo $\cdot \gamma$ - linolenate) zinc oxide, or chelated zinc.

18. A composition according to any preceding claim, comprising further an effective and pharmaceutically acceptable amount of $\alpha$-tocopherol or other antioxidant.

19. A pharmaceutical or dietary pack comprising $\gamma$-linolenic or dihomo - $\gamma$ - linolenic acid or derivative and a material (particularly zinc and/or β-lactam antibiotic) influencing the bodily PG balance, as set out in any preceding claim, presented separately, or two together and one separately, but for conjoint administration.

## Revendications

1 Composition pharmaceutique ou alimentaire comprenant de l'acide $\gamma$-linolénique ou son dérivé physiologiquement fonctionnel et/ou de l'acide dihomo - $\gamma$ - linolénique ou son dérivé physiologiquement fonctionnel et une quantité conjointe d'un antibiotique du type β-lactame, seuls ou dans un véhicule acceptable pour l'usage pharmaceutique ou alimentaire.

2. Composition selon la revendication 1, caractérisée en ce qu l'antibiotique est une pénicilline ou une céphalosporine naturelle ou semi-synthétique.

3. Composition selon la revendication 2, caractérisée en ce que l'antibiotique est choisi parmi la pénicilline G, la pénicilline N, la pénicilline V la céphalothine, l'ampicilline, l'amoxycilline, la cloxacilline, la céphalexine et la céphaloglycine.

4. Composition selon les revendications1, 2 ou 3, comprenant en outre du zinc physiologiquement assimilable.

5. Composition pharmaceutique ou alimentaire pour l'utilisation dans le traitement de la schizophrénia, comprenant de l'acide $\gamma$-linolénique ou son dérivé physiologiquement fonctionnel et/ou de l'acide dihomo $\cdot \gamma \cdot$ linolénique ou son dérivé physiologiquement fonctionnel et une quantité conjointe d'une substance influençant l'équilibre des PG de la série 1/PG de la série 2 dans l'organisme en faveur des PG de la série 1. seuls ou dans un véhicule acceptable pour l'usage pharmaceutique ou alimentaire.

6. Composition pharmaceutique ou alimentaire pour l'utilisation dans le traitement du psoriasis, de l'acné, des pellicules de l'eczéma, de la perte de cheveu (autre que celle due à la calvitie par héritage parternel), comprenant de l'acide $\gamma$-linolénique ou son dérivé physiologiquement fonctionnel et/ou de l'acide dihomo - $\gamma$ - linolénique ou son dérivé physiologiquement fonctionnel et une quantité conjointe d'une substance influençant l'équilibre des PG de la série 1/PG de la série 2 dans l'organisme en faveur des PG de la série 1. seuls ou dans un véhicule acceptable pour l'usage pharmaceutique ou alimentaire.

7. Composition pharmaceutique ou alimentaire pour l'utilisation dans le traitement de l'obésité, comprenant de l'acide $\gamma$-linolénique ou son dérivé physiologiquement fonctionnel et/ou de l'acide dihomo - $\gamma \cdot$ linolénique ou son dérivé physiologiquement fonctionnel, avec une quantité conjointe d'une substance influençant l'équilibre des PG de la série 1/PG de la série 2 dans l'organisme en faveur des PG de la série 1. seuls ou dans un véhicule acceptable pour l'usage pharmaceutique ou alimentaire

8. Composition pharmaceutique ou alimentaire pour l'utilisation dans le traitement des troubles menstruels comprenant les syndromes prémenstruels et la perte de sang excessive pendant les règles ou des règles d'une durée excessive, comprenant de l'acide $\gamma$-linolénique ou son dérivé physiologiquement fonctionnel et/ou de l'acide dihomo $\cdot \gamma$ - linolénique ou son dérivé physiologiquement fonctionnel et une quantité conjointe d'une substance influençant l'équilibre des PG de la série 1/PG de la série 2 dans l'organisme en faveur des PG de la série 1 seuls ou dans un véhicule acceptable pour l'usage pharmaceutique ou alimentaire.

9. Composition selon l'une quelconque des revendications 5 à 8, dans laquelle ladite substance conjointe est du zinc physiologiquement assimilable.

10. Composition selon l'une quelconque des revendications 5 à 8, dans laquelle ladite substance conjointe est un antibiotique du type β-lactame.

11. Composition selon la revendication 10, dans laquelle l'antibiotique est une péniicilline ou une céphalosporine naturelle ou semi-synthétique.

12. Composition selon la revendication 10, dans laquelle l'antibiotique est choisi parmi la pénicilline G, la pénicilline N, la pénicilline V, la céphalothine, l'ampicilline, l'amoxycilline, la cloxacilline, la céphalexine et la céphaloglycine.

13. Composition selon l'une quelconque des revendications 10 à 12, contenant en outre du zinc physiologiquement acceptable.

14. Composition selon l'une quelconque des revendications précédentes dans laquelle le dérivé physiologiquement fonctionnel d'acide $\gamma$-linolénique ou d'acide dihomo - $\gamma$ - linolénique est un ester de méthyle ou d'éthyle ou un glycéride dudit acide.

15. Composition selon l'une quelconque des revendications précédentes, dans laquelle l'acide $\gamma$-linolénique est présent sous forme de l'huile de graine d'Oenothera biennis L, Oenothera lamarckiana, ou d'une autre espèce de primerose du soir, ou une fraction de ladite huile.

16. Composition selon l'une quelconque des revendications précédentes, dans laquelle l'acide $\gamma$-linolénique est présent sous forme de l'huile de graine de Borago officinalis ou d'une autre espèce de borrache, ou une fraction de cette huile.

17 Composition selon l'une quelconque des revendications précédentes spécifiant la présence de zinc, dans laquelle le zinc est présent sous forme d'un sel d'un acide inorganique ou organique (et en particulier d'oléate de zinc, de $\gamma$-linolénate de zinc ou de dihomo - $\gamma$ - linolénate de zinc), d'oxyde de zinc ou de zinc chélaté.

18. Composition selon l'une quelconque des revendications précédentes, contenant en outre une quantité efficace et pharmaceutiquement acceptable d'$\alpha$-tocophérol ou d'un autre antioxydant.

19. Emballage pharmaceutique ou alimentaire comprenant de l'acide $\gamma$-linolénique ou de l'acide dihomo - $\gamma$ - linolénique ou leurs dérivés et une substance (en particulier du zinc et/ou un antibiotique du type β-lactame) influençant l'équilibre des PG dans l'organisme, comme indiqué dans l'une quelconque des revendications précédentes, présentées séparément ou deux ensemble et une séparément, mais pour l'administration conjointe.

**Patentansprüche**

1. Pharmazeutische oder diätetische Zusammensetzung, die $\gamma$-Linolensäure oder ein physiologisch funktionelles Derivat davon und/oder Dihomo - $\gamma$ - linolensäure oder ein physiologisch funktionelles Derivat davon, und eine zur gemeinsamen Verwendung geeignete Menge eines β · Lactam - Antibiotikums allein oder mit einer pharmazeutisch oder diätetisch verträglichen Trägersubstanz enthält.

2. Zusammensetzung nach Anspruch 1 dadurch gekennzeichnet, daß das Antibiotikum ein natürliches oder halb-synthetisches Penicillin- oder Cephalosporin-Antibiotikum ist.

3 Zusammensetzung nach Anspruch 2, dadurch gekennzeichnet, daß das Antibiotikum ein solches aus der Gruppe Penicillin G, Penicillin N, Penicillin V, Cephalothin, Ampicillin, Amoxycillin, Cloxacillin, Cephalexin und Cephaloglycin ist.

4. Zusammensetzung nach Anspruch 1, 2 oder 3, dadurch gekennzeichnet, daß sie zusätzlich physiologisch assimilierbares Zink enthält.

5. Pharmazeutische oder diätetische Zusammensetzung zur Verwendung bei der Behandlung von Schizophrenie, dadurch gekennzeichnet, daß sie $\gamma$-Linolensäure oder ein physiologisch funktionelles Derivat davon und/oder Dihomo · $\gamma$ - linolensäure oder ein physiologisch funktionelles Derivat davon und eine zur gemeinsamen Verwendung geeignete Menge eines Stoffes enthält, der das 1 - Serien/2 - Serien Prostaglandin (PG)-Gleichgewicht im Körper zu Gunsten der 1 - Serien - Prostaglandine beeinflußt, allein oder mit einer pharmazeutisch oder diätetisch verträglichen Trägersubstanz.

6. Pharmazeutische oder diätetische Zusammensetzung zur Verwendung bei der Behandlung von Psoriasis, Akne, Kopfschuppen, Ekzemen oder Haarausfall (einer anderen Art als die der vererbten Veranlagung zur männlichen Kahlköpfigkeit), dadurch gekennzeichnet, daß sie $\gamma$-Linolensäure oder ein physiologisch funktionelles Derivat davon und/oder Dihomo - $\gamma$ - linolensäure oder ein physiologisch funktionelles Derivat davon und eine zur gemeinsamen Verwendung geeignete Menge eines Stoffes enthält, der das 1 - Serien/2 - Serien - Prostaglandin · Gleichgewicht im Körper zu Gunsten der 1 - Serien - Prostaglandine beeinflußt, allein oder mit einer pharmazeutisch oder diätetisch verträglichen Trägersubstanz.

7 Pharmazeutische oder diätetische Zusammensetzung zur Verwendung bei der Behandlung von Fettsucht, dadurch gekennzeichnet, daß sie $\gamma$-Linolensäure oder ein physiologisch funktionelles Derivat davon und/oder Dihomo - $\gamma$ - linolensäure oder ein physiologisch funktionelles Derivat davon zusammen mit einer zur gemeinsamen Verwendung geeigneten Menge eines Stoffes enthält, der das 1 - Serien/2 - Serien - Prostaglandin - Gleichgewicht im Körper zu Gunsten der 1 - Serien - Prostaglandine beeinflußt, allein oder mit einer pharmazeutisch oder diätetisch verträglichen Trägersubstanz.

8. Pharmazeutische oder diätetische Zusammensetzung zur Verwendung bei der Behandlung von Menstruationsstörungen einschließlich premenstrueller Syndrome und übermäßigem Blutverlust während der Menstruation oder der Dauer der Menstruation, dadurch gekennzeichnet, daß sie $\gamma$-Linolensäure oder ein physiologisch funktionelles Derivat davon und/oder Dihomo - $\gamma$ - linolensäure oder ein physiologisch funktionelles Derivat davon und eine zur gemeinsamen Verwendung geeignete Menge eines Stoffes enthält, der das 1 - Serien/2 - Serien - Prostaglandin - Gleichgewicht im Körper zu Gunsten der 1 - Serien - Prostaglandine beeinflußt, allein oder mit einer pharmazeutisch oder diätetisch verträglichen Trägersubstanz.

9. Zusammensetzung nach einem der Ansprüche 5 bis 8, dadurch gekennzeichnet, daß der gemeinsam verwendete Stoff physiologisch assimilierbares Zink ist.

10. Zusammensetzung nach einem der Ansprüche 5 bis 8, dadurch gekennzeichnet, daß der gemeinsam verwendete Stoff ein $\beta$ - Lactam - Antibiotikum ist.

11. Zusammensetzung nach Anspruch 10, dadurch gekennzeichnet, daß das Antibiotikum ein natürliches oder halbsynthetisches Penicillin- oder Cephalosporin-Antibiotikum ist.

12. Zusammensetzung nach Anspruch 10, dadurch gekennzeichnet, daß das Antibiotikum ein solches aus der Gruppe Penicillin G, Penicillin N, Penicillin V, Cephalothin, Ampicillin, Amoxycillin, Cloxacillin, Cephalexin und Cephaloglycin ist.

13. Zusammensetzung nach einem der Ansprüche 10 bis 12, dadurch gekennzeichnet, daß sie zusätzlich physiologisch assimilierbares Zink enthält.

14. Zusammensetzung nach einem der vorhergehenden Ansprüche, dadurch gekennzeichnet, daß das physiologisch funktionelle Derivat der $\gamma$-Linolensäure oder Dihomo - $\gamma$ - linolensäure ein Methyl- oder Äthylester oder Glycerid davon ist.

15. Zusammensetzung nach einem der vorangehenden Ansprüche, dadurch gekennzeichnet, daß die $\gamma$-Linolensäure als Öl von Samen von Oenothera biennis L, Oenothera lamarckiana oder anderen Nachtkerzen-Arten, oder einer Fraktion davon, vorliegt.

16. Zusammensetzung nach einem der vorangehenden Ansprüche, dadurch gekennzeichnet, daß die $\gamma$-Linolensäure als Öl von Samen von Borago officinalis oder anderen Boretsch-Arten, oder einer Fraktion davon, vorliegt.

17. Zusammensetzung nach einem der vorangehenden Ansprüche, die die Gegenwart von Zink nennen, dadurch gekennzeichnet, daß das Zink als Salz einer Mineralsäure oder organischen Säure (und insbesondere als Zinkoleat, Zink - $\gamma$ - linolenat oder Zink - dihomo - $\gamma$ - linolenat), Zinkoxid, oder als chelatisiertes Zink vorliegt.

18. Zusammensetzung nach einem der vorangehenden Ansprüche, dadurch gekennzeichnet, daß sie außerdem eine wirksame und pharmazeutisch verträgliche Menge von $\alpha$-Tocopherol oder einem anderen Antioxidans enthält.

19. Packung zur pharmazeutischen oder diätetischen Verwendung, die $\gamma$-Linolensäure oder Dihomo - $\gamma$ - linolensäure oder ein Derivat davon und einen das Prostaglandin-Gleichgewicht im Körper beeinflussenden Stoff (insbesondere Zink- und/oder ein $\beta$ - Lactam - Antibiotikum) nach einem der vorangehenden Ansprüche, voneinander getrennt, oder zwei Stoffe zusammen und einen davon getrennt, aber zur gemeinsamen Verabreichung geeignet, enthält.